(19) Europäisches Patentamt
European Patent Office
Office européen des brevets

(11) Numéro de publication: **0 211 698**
**B1**

(12) # FASCICULE DE BREVET EUROPEEN

(45) Date de publication du fascicule du brevet: **30.01.91**

(51) Int. Cl.$^5$: **C 07 D 403/04,** A 61 K 31/415

(21) Numéro de dépôt: **86400200.1**

(22) Date de dépôt: **31.01.86**

(54) **Dérivés de (dihydro-4,5 1H-imidazolyl-2)-2 dihydro-2,3 indole, leur préparation et leur application en thérapeutique.**

(30) Priorité: **08.02.85 FR 8501774**
**08.02.85 FR 8501775**

(43) Date de publication de la demande:
**25.02.87 Bulletin 87/09**

(45) Mention de la délivrance du brevet:
**30.01.91 Bulletin 91/05**

(84) Etats contractants désignés:
**AT BE CH DE FR GB IT LI LU NL SE**

(56) Documents cités:
**EP-A-0 054 147**
**EP-A-0 077 024**
**EP-A-0 141 686**

(73) Titulaire: **SYNTHELABO**
**58, rue de la Glacière**
**F-75621 Paris Cedex 13 (FR)**

(72) Inventeur: **Bigg, Denis**
**5, Parc de Diane**
**F-78350 Jouy-en-Joses (FR)**
Inventeur: **Maloizel, Christian**
**21, Rue du Plateau**
**F-92190 Meudon (FR)**
Inventeur: **Menin, Jacques**
**Cité Balzac 118, Rue de Balzac D. 114**
**F-94400 Vitry-sur-Seine (FR)**
Inventeur: **Merly, Jean**
**68, Rue d'Estienne d'Orves**
**F-92260 Fontenay-aux-Roses (FR)**

(74) Mandataire: **Ludwig, Jacques et al**
**SYNTHELABO, Service Brevets, 22, avenue Galilée, B.P. 72**
**F-92352 Le Plessis Robinson Cédex (FR)**

**EP 0 211 698 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention à pour objet des dérivés de (dihydro-4,5 1*H*-imidazolyl-2)-2 dihydro-2,3 indole, leur préparation et leur application en thérapeutique.

Les composés de l'invention répondent à la formule générale (I)

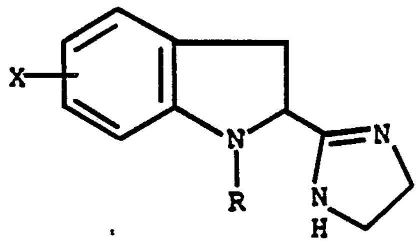

(I)

dans laquelle

X est en position 4, 5, 6 ou 7 et représente un atome d'halogène, un radical $(C_{1-4})$alcoxy, un radical $(C_{1-4})$alkyle ou le radical allyloxy, ou encore, lorsque R représente un radical phényle éventuellement substitué, X peut représenter aussi un atome d'hydrogène et

R représente un radical $(C_{1-6})$alkyle droit ou ramifié, un radical $(C_{3-6})$cycloalkyl-$(C_{1-4})$alkyle, un radical $(C_{3-6})$alcényle, un radical aryl-$(C_{1-4})$alkyle pouvant porter un ou plusieurs substituants choisis parmi les atomes d'halogène et les radicaux méthyle, méthoxy et méthylènedioxy, ou encore un radical phényle éventuellement substitué, de formule

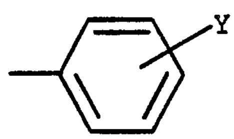

dans laquelle Y représente un atome d'hydrogène ou d'halogène, ou un radical $(C_{1-6})$alkyle droit ou ramifié ou un radical $(C_{1-6})$alcoxy droit ou ramifié.

Les composé de l'invention peuvent exister sous la forme de racémates ou d'énantiomères qui font partie de l'invention. Les sels pharmaceutiquement acceptables des composés (I) font également partie de l'invention.

Une première série de composés préférés est celle dans la formule (I) desquels X est un atome de chlore ou de fluor ou un radical méthyle, méthoxy ou allyloxy (plus particulièrement en position 4, 6 ou 7), et R est un radical alkyle, benzyle, allyle ou cyclopropylméthyle.

Une autre série de composés préférés est celle dans la formule (I) desquels X est un atome d'hydrogène ou un radical méthyle (plus particulièrement en position 4) et R est un radical phényle non substitué ou portant un radical méthyle, éthyle, propyle, méthoxy, éthoxy ou propoxy (plus particulièrement en position 3 ou 4).

Les composés de l'invention dans la formule (I) desquels R représente un radical alkyle, cycloalkylalkyle, alcényle ou arylalkyle, peuvent être préparés selon le schéma réactionnel 1 donné ci-après.

Les esters de départ (II) sont décrits dans la littérature. On hydrogène l'ester de départ (II) dans lequel R' est un radical $(C_{1-4})$alkyle à l'aide de gaz chlorhydrique et d'étain.

On fait ensuite réagir l'ester (III) et un composé RZ (Z étant un groupe labile, en particulier un atome d'iode ou de brome) dans un solvant tel que l'acétone, la méthyléthylcétone ou le diméthylformamide, à la température ambiante ou à température plus élevée, en présence d'une base, telle que le carbonate de potassium. La réaction peut être catalysée par exemple par addition d'iodure de sodium.

La formation de l'imidazoline à partir de l'ester (IV) est effectuée par l'éthylènediamine en présence de triméthylaluminium.

Schéma réactionnel 1

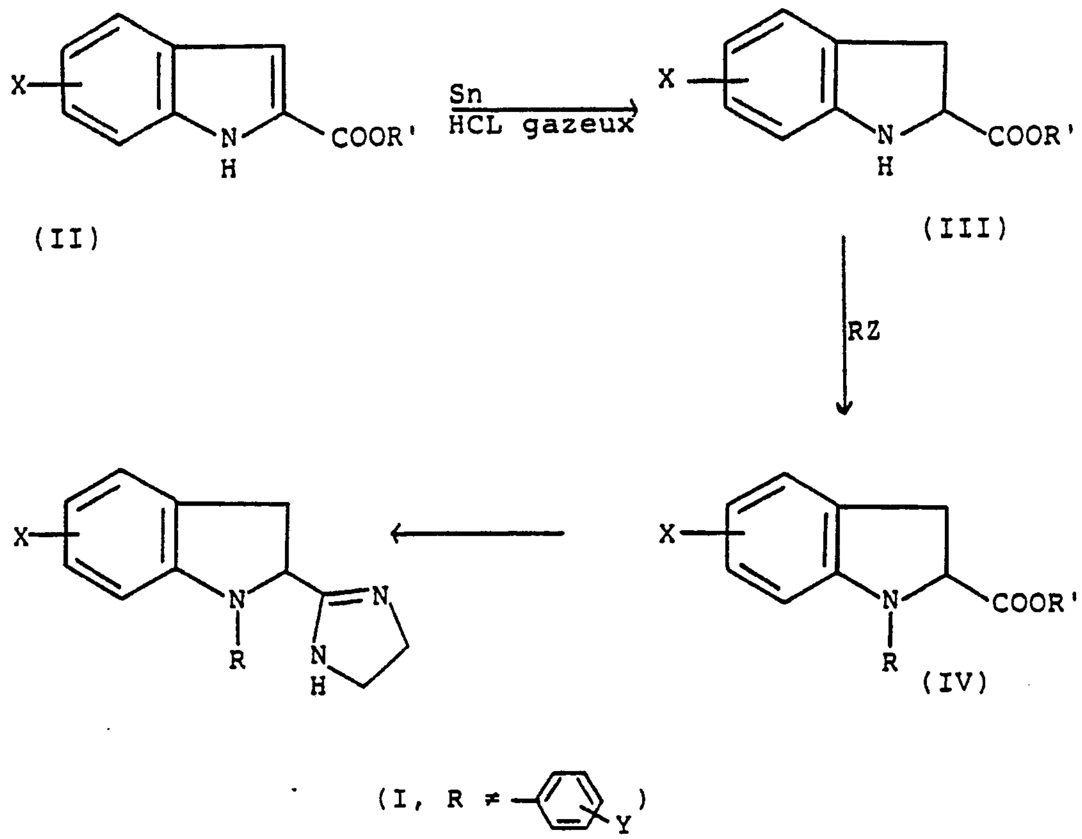


(II) (III) (IV)

(I, R ≠ )

Les composés de l'invention dans la formule (I) desquels R représente un radical phényle portant un substituant Y peuvent être préparés selon le schéma réactionnel 2 donné ci-après.

Les acides de départ (V) sont décrits dans la littérature. On les fait réagir avec de l'ammoniac en présence de carbonyldiimidazole pour obtenir l'amide (VI) que l'on réduit à l'aide d'ammoniac liquide et de sodium, puis on transforme l'amide (VII) en nitrile (VIII) à l'aide de chlorure de p-toluènesulfonyle en présence de pyridine, ou par toute autre méthode connue, et l'on fait réagir le nitrile (VIII) avec de l'éthylènediamine pour obtenir le composé (I).

Enfin, lorsque X représente l'hydrogène et R représente un radical phényle substitué ou non, le composé peut encore être préparé selon le schéma réactionnel 3 (cf. exemple 3).

Les exemples suivants illustrent l'invention. Les spectres IR et RMN et les analyses confirment la structure des composés.

Exemple 1

(dihydro-4,5, 1*H*-imidazolyl-2)-2 benzyl-1 chloro-4 dihydro-2,3 1*H*-indole et son fumarate

1.1. Chloro-4 dihydro-2,3 1*H*-indolecarboxylate-2 d'éthyle

Dans un ballon tricol de 2 l, que l'on maintient à −50°C dans un mélange de carboglace et d'isopropanol, on introduit 350 ml d'éthanol et on fait passer un courant de gaz chlorhydrique.

On introduit alors 33,0 g (0,148 mole) de chloro-4 1*H*-indolecarboxylate-2 d'éthyle puis on ajoute 105,4 g (0,888 at-g) d'étain en une seule fois. On agite le mélange réactionnel et le laisse revenir lentement à la température ambiante. On agite la solution pendant 40 h et la traite par un courant d'ammoniac. On filtre le précipité formé et le rince à l'alcool. On concentrè la phase alcoolique et la reprend par du dichlorométhane.

Après lavage à l'eau, séchage et concentration, on obtient une huile orange que l'on reprend par du cyclohexane. On obtient un solide. F = 48, 5—50, 5°C.

Schéma réactionnel 2

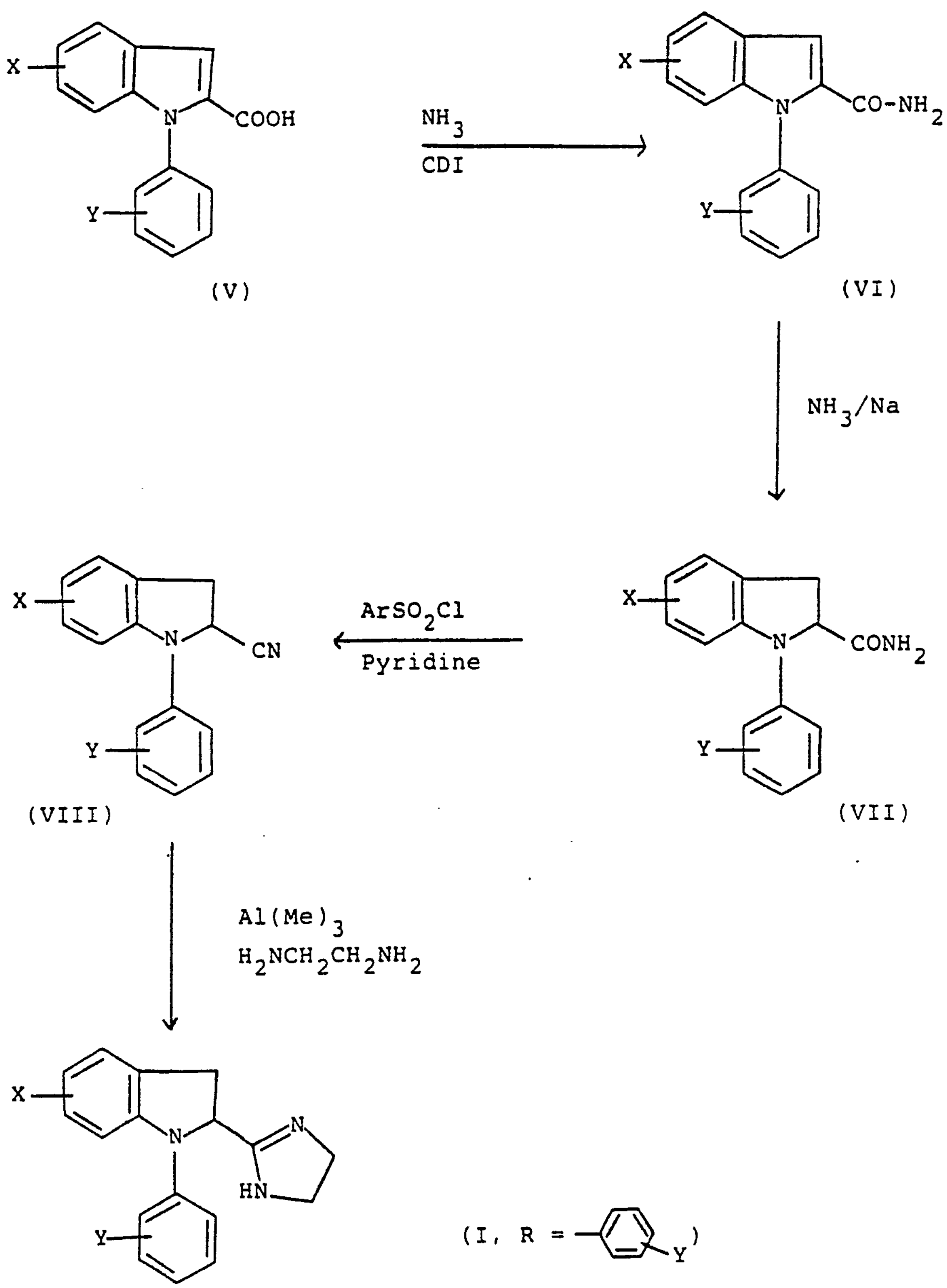


(V) (VI) (VII) (VIII)

(I, R = phényl-Y)

Schéma réactionnel 3

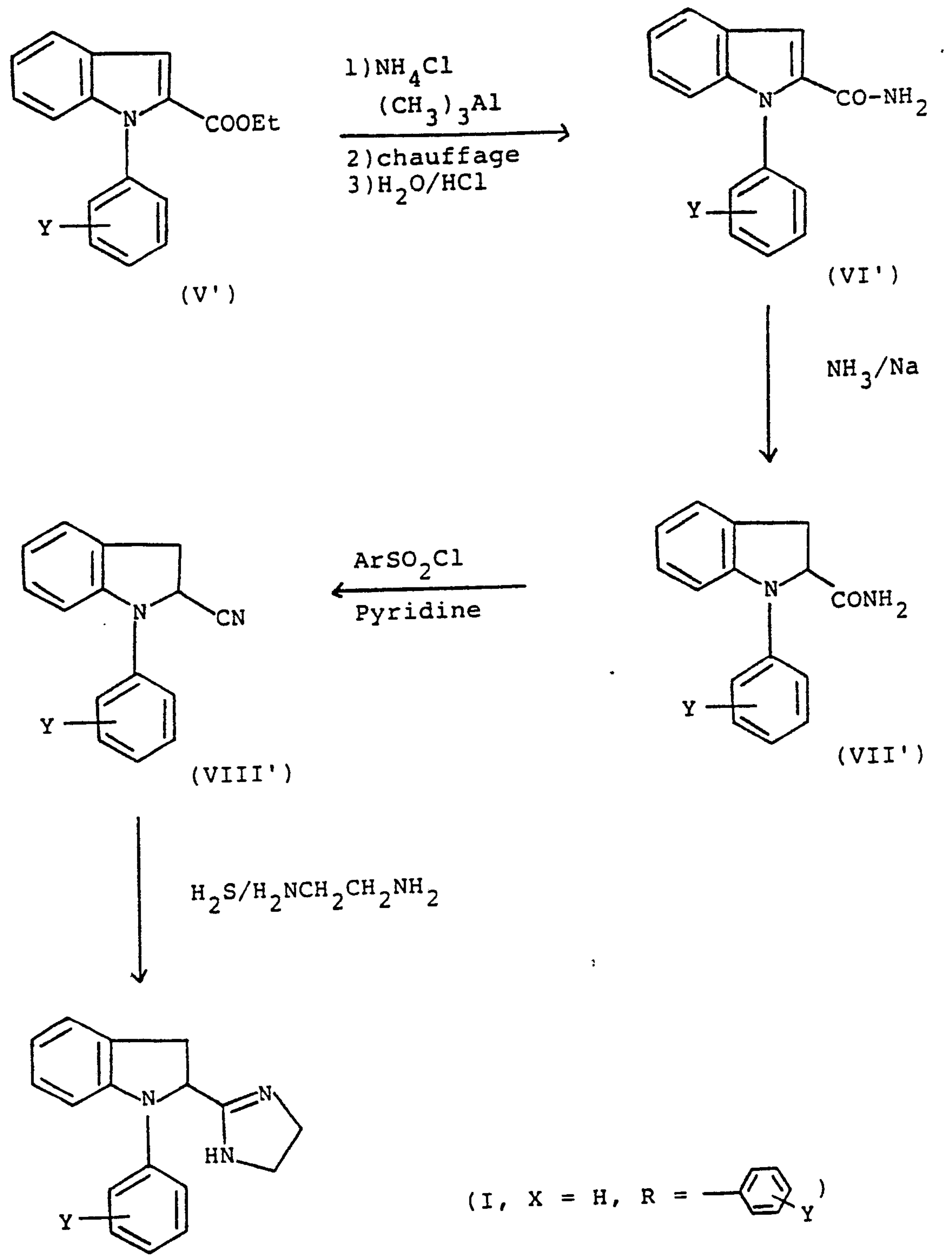


1.2. Benzyl-1 chloro-4 dihydro-2,3 1*H* indolecarboxylate-2 d'éthyle

Dans un ballon de 500 ml, on introduit, sous argon, 7,9 g (0,035 mole) de l'ester obtenu sous 1., 7,7 g (0.056 mole) de $K_2CO_3$ et 100 ml de diméthylformamide puis 7,2 g (0,042 mole) de bromure de benzyle.

On agite le mélange réactionnel à la température ambiante pendant 24 h. On le verse sur un mélange d'eau et de glace, extrait à l'éther, lave à l'eau, sèche et concentre. On obtient une huile que l'on purifie par chromatographie sur silice à l'aide de l'éluant cyclohexane/acétate d'éthyle (99/1) et que l'on utilise telle qu'elle dans l'étape suivante.

1.3. (dihydro-4,5 1*H*-imidazolyl-2)-2 benzyl-1 chloro-4 dihydro-2,3 1*H*-indole et son fumarate

A une solution de 2,9 g (0,0403 mole) de triméthylaluminium (16,5 ml d'une solution à 25% dans de l'hexane) dans 30 ml de toluène, refroidie à 0°C, on ajoute, sous argon, goutte à goutte, en 30 mn, une solution de 2,46 g (0,0410 mole) d'éthylènediamine dans 15 ml de toluène.

On porte ce mélange réactionnel à 50°C et ajoute alors 5,3 g (0,0168 mole) de l'ester obtenu sous 2. en solution dans 30 ml de toluène. On chauffe le mélange à 90°C, distille l'hexane et porte à la température du reflux pendant 4 h. On hydrolyse par addition d'eau et refroidit. On filtre la solution, rince avec $CH_2CL_2$, extrait avec $CH_2Cl_2$, lave à l'eau, sèche et concentre. On obtient un solide blanc.

On transforme le composé obtenu en fumarate en faisant réagir 4,6 g (0,0148 mole) de la base en solution dans 50 ml d'éthanol et 1,63 g (0,0140 mole) d'acide fumarique en solution dans 100 ml d'éthanol.

Un solide blanc se forme qui après filtration et recristallisation dans le méthanol fond à 229—231°C.

Exemple 2

(dihydro-4,5 1*H*-imidazolyl-2)-2 méthyl-4 phényl-1 dihydro-2,3 1*H*-indole et son fumarate

2.1. méthyl-4 phényl-1 1*H*-indolecarboxamide-2

A une solution de 10,9 g (0,0434 mole) d'acide méthyl-4 phényl-1 indolecarboxylique-2 dans 500 ml de $CH_2Cl_2$, on ajoute, par positions, 10,95 g (0,0675 mole) de carbonyldiimidazole.

On agite le mélange réactionnel pendant 3 h à la température ambiante. On le refroidit ensuite dans un mélange d'eau et de glace et fait passer un courant d'ammoniac. Après avoir fait barboter l'ammoniac pendant 1 h, on agite le mélange réactionnel à la température ambiante pendant 22 h. On le reprend par de l'eau et élimine le précipité. On lave la phase organique, la sèche et la concentre. On obtient un solide que l'on fait recristalliser dans un mélange toluène/acétate d'éthyle (1/1).

F = 187—189°C.

2.2. méthyl-4 phényl-1 dihydro-2,3 1*H*-indolecarboxamide-2

Dans un mélange de 100 ml de tétrahydrofuranne et de 200 ml d'ammoniac liquide, on ajoute 8,6 g (0,0344 mole) de l'amide obtenu sous 1., puis on introduit 1,74 g (0,0757 at-g) de sodium en 15 mn. On agite le mélange réactionnel pendant 30 mn. On l'hydrolyse par 100 ml d'une solution de $NH_4Cl$, laisse décanter et extrait à l'acétate d'éthyle, lave à l'eau, sèche et concentre.

Après recristallisation dans un mélange toluène/acétate d'éthyle (1/2) on obtient un solide blanc.

F = 187—189°C.

2.3. Méthyl-4 phényl-1 dihydro-2,3 1*H*-indolecarbonitrile-2

Dans un ballon tricol de 250 ml, on introduit 7,0 g (0,0277 mole) de l'amide obtenu sous 2. et 9,9 g (0,125 mole) de pyridine. On ajoute lentement 10,6 g (0,0555 mole) de chlorure de p-toluènesulfonyle. On agite le mélange réactionnel pendant 23 h à la température ambiante. On le verse sur un mélange d'eau et de glace, extrait à l'éther, lave à l'eau acidulée (5% HCl en poids), puis à l'eau, sèche et concentre à froid. On reprend par du cyclohexane l'huile obtenue. Un solide blanc cristallise.

F = 98,5—100,5°C.

2.4. (dihydro-4,5 1*H*-imidazolyl-2)-2 méthyl-4 phényl-1 dihydro-2,3 1*H*-indole et son fumarate

A une solution de 4,06 g (0,0563 mole) de triméthylaluminium (23,0 ml d'une solution à 25% dans de l'hexane) dans 50 ml de toluène, on ajoute, sous argon, à 0—5°C, goutte à goutte, en 30 mn, une solution de 3,4 g (0,0569 mole) d'éthylènediamine dans 20 ml de toluène.

On porte le mélange réactionnel à 50°C et ajoute 5,5 g (0,0235 mole) du nitrile obtenu sous 3. en solution dans 50 ml de toluène.

L'addition terminée, on chauffe le mélange à 90°C, distille l'hexane et porte à la température du reflux pendant 10 h. On hydrolyse le mélange, tout en le refroidissant par 60 ml d'eau. On filtre la solution obtenue et rince avec $CH_2Cl_2$. On décante la phase organique, lave à l'eau, sèche et concentre.

On obtient un solide blanc.

On prépare le fumarate en faisant réagir 3,55 g (0,0128 mole) de base en solution dans 100 ml d'éthanol avec 1,4 g (0,0122 mole) d'acide fumarique en solution dans 100 ml d'éthanol.

On obtient une huile que l'on triture dans de l'acétone. Le solide est recristallisé dans le l'alcool isopropylique.

F = 199—200,5°C.

Exemple 3

(dihydro-4,5 1*H*-imidazolyl-2)-2 phényl-1 dihydro-2,3 1*H*-indole

3.1. Phényl-1 1*H*-indolecarboxamide-2

A une suspension de 5,35 g (0,10 mole) de chlorure d'ammonium dans 100 ml de benzène sec, à 5°C, on ajoute lentement 41,5 ml d'une solution de triméthylaluminium à 25% dans de l'hexane. On rince l'ampoule avec 10 ml de benzène sec. On laisse le mélange réactionnel revenir à la température ambiante, puis on agite pendant 2 h.

A une solution de 8,0 g (0,03 mole) de phényl-1 1*H*-indolecarboxylate-2 d'éthyle dans 300 ml de benzène, on ajoute 135 ml (0,09 mole) du réactif préparé précédement. On chauffe le mélange réactionnel à

50°C, sous argon, pendant 12 h. On hydrolyse par 50 ml de HCl à 5%, filtre, lave à l'acétate d'éthyle. Les phases organiques sont lavées à l'eau, séchées et concentrées. On obtient un solide que l'on lave au toluène et fait recristalliser dans de l'acétone.

F = 193—194,5°C.

3.2. Phényl-1 dihydro-2,3 1*H*-indolecarboxamide-2

Dans un mélange de 175 ml de THF sec et de 175 ml d'ammoniac liquide, on ajoute 9,4 g (0,0398 mole) de phényl-1 1*H*-indolecarboxamide-2 et ajoute 2,0 g (0,088 atome-gramme) de sodium, en 10 mn, puis on agite 30 mn. On hydrolyse par 100 ml d'une solution de $NH_4Cl$, décante, extrait à l'acétate d'éthyle, lave à l'eau, sèche et concentre. Le solide obtenu est recristallisé dans du toluène.

F = 190—191,5°C.

3.3. Phényl-1 dihydro-2,3 1*H*-indolecarbonitrile-2

Dans un tricol de 250 ml, on introduit 6,0 g (0,025 mole) de phényl-1 dihydro-2,3 1*H*-indole-carboxamide-2 et 9,0 g (9,2 ml, 0,113 mole) de pyridine. On ajoute lentement 9,5 g (0,050 mole) de chlorure de p-toluènesulfonyle et agite le mélange réactionnel 3h30, à la température ambiante. On verse le mélange réactionnel sur de l'eau et de la glace, extrait à l'éther, lave avec de l'acide chlorhydrique à 5%, puis à l'eau, sèche et concentre à froid. On reprend l'huile formée dans du cyclohexane. On concentre la phase cyclohexanique, on obtient un solide.

F = 47,5—49°C.

3.4. (dihydro-4,5 1*H*-imidazolyl-2)-2 phényl-1 dihydro-2,3 1*H*-indole

On sature à 0°C, par $H_2S$, une solution de 2,2 g (0,01 mole) de phényl-1 dihydro-2,3, 1*H*-indole-carbonitrile-2 dans 1,8 g (2,06 ml, 0,03 mole) d'éthylènediamine. On laisse reposer à la température ambiante pendant 4 jours. On reprend le solide formé par $CH_2CL_2$, lave 3 fois à l'eau, sèche et concentre. On obtient une huile.

On prépare le fumarate du composé obtenu en faisant réagir 3,5 g (0,0133 mole) de base en solution dans 50 ml d'éthanol et 1,4 g (0,0120 mole) d'acide fumarique en solution dans 80 ml d'éthanol. On concentre la solution, reprend le concentré par de l'acétate d'éthyle et triture; on obtient un solide que l'on fait recristalliser dans de l'éthanol.

F = 212,5—215°C.

Le tableau ci-après illustre les structures et propriétés physiques de quelques composés selon l'intervention.

Tableau

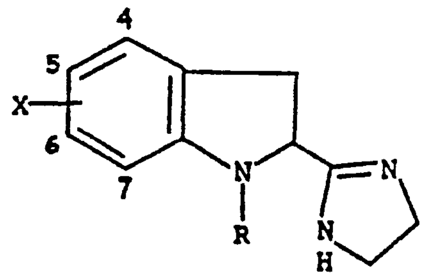

| Composé | X | R | Sel ou base (*) | F(°C) |
|---|---|---|---|---|
| 1 | 4-Cl | benzyl | 08 | 229-231 |
| 2 | 4-Cl | allyl | 08 | 200-201,5 |
| 3 | 4-Cl | n-propyl | 08 | 179,5-181 |
| 4 | 4-Cl | cyclopropylméthyl | 08 | 202-203,5 |
| 5 | 4-MeO | benzyl | 08 | 198-199 |
| 6 | 6-F | benzyl | 08 | 174-176 |
| 7 | 6-Me | benzyl | 08 | 159-161 |
| 8 | 6-Me | allyl | 08 | 184-185 |
| 9 | 6-Me | cyclopropylméthyl | 08 | 178-180 |
| 10 | 4-allyloxy | benzyl | 00 | 154-155 |
| 11 | 4-allyloxy | n-butyl | 08 | 115-117 |
| 12 | 4-allyloxy | n-pentyl | 00 | 102-103 |
| 13 | 5-MeO | benzyl | 08 | 152-154,5 |
| 14 | 5-MeO | éthyl | 00 | 130-132 |
| 15 | 5-MeO | isobutyl | 00 | 153-154,5 |
| 16 | 6-allyloxy | benzyl | 10 | 149-152 |
| 17 | 6-n-propyloxy | benzyl | 10 | 161-164 |
| 18 | 4-Me | benzyl | 10 | 201-204 |
| 19 | 4-Me | allyl | 10 | 187-189 |
| 20 | 7-Cl | allyl | 10 | 219-221 |
| 21 | 7-Cl | benzyl | 10 | 209-211 |
| 22 | 7-F | allyl | 10 | 175-177 |
| 23 | 7-F | benzyl | 10 | 104-108 |

| Composé | X | R | Sel ou base (*) | F(°C) |
|---|---|---|---|---|
| 24 | 7-F | cyclopro-pylméthyl | 10 | 212-214 |
| 25 | 4-F | allyl | 10 | 189-192 |
| 26 | 4-F | benzyl | 10 | 187-189 |
| 27 | 4-F | cyclopro-pylméthyl | 10 | 169-171 |
| 28 | 4-F | n-propyl | 10 | 170-172 |
| 29 | 4-F | n-pentyl | 10 | 173-175 |
| 30 | 4-F | n-hexyl | 10 | 164-166 |
| 31 | 4-Cl | 2-Cl-benzyl | 10 | 184 |
| 32 | 4-Cl | 2-MeO-benzyl | 10 | 204-206 |
| 33 | 4-Cl | 2-F-benzyl | 10 | 192-193 |
| 34 | 4-Cl | 4-Me-benzyl | 10 | 258-262 |
| 35 | 4-Cl | 2-Me-benzyl | 10 | 214-218 |
| 36 | H | phényl | 08 | 212,5-215 |
| 37 | H | 4-Me-phényl | 08 | 199-201 |
| 38 | H | 3-MeO-phényl | 08 | 157-160 |
| 39 | H | 4-MeO-phényl | 08 | 89-93(**) |
| 40 | H | 3-Me-phényl | 08 | 174-175 |
| 41 | 4-Me | phényl | 08 | 199-200,5 |
| 42 | 4-Me | 4-MeO-phényl | 00 | 169,5-171,5 |
| 43 | H | 4-Et-phényl | 08 | 127-130 |
| 44 | H | 4-EtO-phényl | 08 | 124-127 |
| 45 | H | 3-EtO-phényl | 08 | 151-153 |
| 46 | H | 3-PrO-phényl | 08 | 157-159 |
| 47 | H | 3-Et-phényl | 08 | 158-161,5 |
| 48 | H | 3-Pr-phényl | 08 | 135-137 |
| 49 | 5-MeO | phényl | 08 | 194-196 |

(*) 00 : base
08 : fumarate
10 : chlorhydrate

(**) hémi-hydrate

Les composés de l'invention ont été soumis à des essais pharmacologiques qui ont montré leur intérêt en tant que $\alpha_2$-antagonistes.

A cet effet les composés ont été étudiés dans le test de potentialité et de sélectivité des antagonistes à l'égard des récepteurs $\alpha_2$ in vitro.

La détermination de la valeur $pA_2$ à l'égard des effets inhibiteurs de la clonidine, $\alpha_2$-agoniste bien connu, a eu lieu sur le vas deferens du rat stimulé à une fréquence de 0,1 Hz en présence de 300 nM de prazosine et de 1 µM de cocaïne, selon la méthode décrite par G. M. Drew (European Journal of Pharmacology, 42, (1977) 123—130).

Les $pA_2$ des composés de l'invention sont compris entre 6 et 11.

Les composés de l'invention sont des $\alpha_2$-antagonistes puissants qui peuvent être utilisés pour le traitement de la dépression (soit seuls, soit en association avec un produit qui inhibe les mécanismes de captation neuronale), le traitement de l'hypotension, le traitement de l'ileum paralytique post-opératoire, le traitement de l'asthme et de l'obésité, le traitement du diabète, le traitement de l'impuissance.

Par ailleurs certains des composés de l'invention sont également des $\alpha_1$-agonistes qui présentent de l'intérêt comme vasoconstricteurs nasals.

Certains des composés sont des substances qui contractent puissamment in vitro, une préparation d'artère pulmonaire de lapin (préparation contenant au niveau post synaptique seulement des récepteurs $\alpha_1$), selon Starke et al, 1975, Naunyn Schmiedeberg's Arch. Pharmacol., 291, 55—78.

La concentration de composé (I) nécessaire pour obtenir 50% de la contraction maximum (CE 50) va de 10 µM à 0,1 µM.

Ces composés sont bien des agonistes des récepteurs $\alpha_1$, car les contractions qu'ils induisent sur la préparation d'artère pulmonaire de lapin sont antagonisées par la prazosine, antagoniste des récepteurs $\alpha_1$.

Ils peuvent donc être utilisés en thérapeutique par exemple comme vasoconstricteurs nasals.

Les compositions pharmaceutiques à activités $\alpha_2$-antagonistes peuvent être sous forme appropriée pour l'administration par voie orale, rectale ou parentérale; par exemple sous la forme de capsules, comprimés, granulés, gélules ou solutés liquides, sirops ou suspensions buvables, et contenir les excipients appropriés.

Les compositions pharmaceutiques à activités $\alpha_1$-agonistes, utilisables pour le traitement des congestions nasales, peuvent être sous une forme appropriée pour l'administration par voie externe ou locale, par exemple sous la forme d'aérosols ou de solutions pulvérisables, et contenir les excipients appropriés.

La posologie quotidienne pêut aller de 0,1 à 10 mg/kg p.o.

**Revendications pour les Etats contractants: BE CH DE FR GB IT LI LU NL SE**

1. Composés sous forme de racémates ou d'isomères optiquement actifs, caractérisés en ce qu'ils répondent à la formule générale (I)

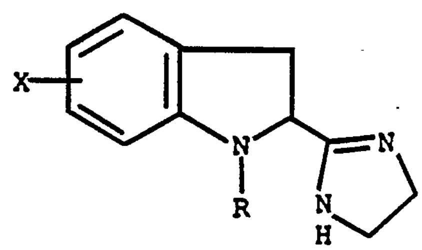

(I)

dans laquelle

X est en position 4, 5, 6 ou 7 et représente un atome d'halogène, un radical $(C_{1-4})$alcoxy, un radical $(C_{1-4})$alkyle ou le radical allyloxy, ou encore, lorsque $R^-$ représente un radical phényle éventuellement substitué, X peut représenter aussi un atome d'hydrogène, et

R représente un radical $(C_{1-6})$alkyle droit ou ramifié, un radical $(C_{3-6})$cycloalkyl-$(C_{1-4})$alkyle, un radical $(C_{3-6})$alcényle, un radical aryl-$(C_{1-4})$alkyle pouvant porter un ou plusieurs substituants choisis parmi les atomes d'halogène et les radicaux méthyle, méthoxy et méthylènedioxy, ou encore un radical phényle éventuellement substitué, de formula

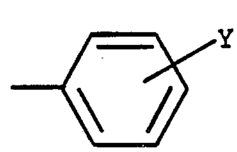

dans laquelle Y représente un atome d'hydrogène ou d'halogène, ou un radical $(C_{1-6})$alkyle droit ou ramifié

ou un radical ($C_{1-6}$)alcoxy droit ou ramifié, ainsi que leurs sels d'addition à des acides pharmaceutiquement acceptables.

2. Composés selon la revendication 1, caractérisés en ce que X représente un atome de chlore ou de fluor ou un radical méthyle, méthoxy ou allyloxy, et R représente un radical alkyle, benzyle, allyle ou cyclopropylméthyle.

3. Composés selon la revendication 2, caractérisé en ce que X est en position 4, 6 ou 7.

4. Composés selon la revendication 1, caractérisés en ce que X représente un atome d'hydrogène ou un radical mèthyle, et R représente un radical phényle, méthylphényle, éthylphényle, méthoxyphényle, éthoxyphényle ou propoxyphényle.

5. Composés selon la revendication 4, caractérisés en ce que X est en position 4 et R représente un groupe phényle substitué en position 3 ou 4.

6. Procédé de préparation des composés selon la revendication 1, dans la formule (I) desquels R représente un radical alkyle, cycloalkylalkyle, acényle ou arylalkyl, procédé caractérisé en ce qu'on hydrogène un ester de formule (II)

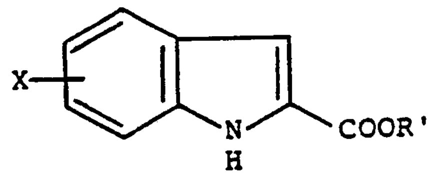

(II)

en ester de formule (III)

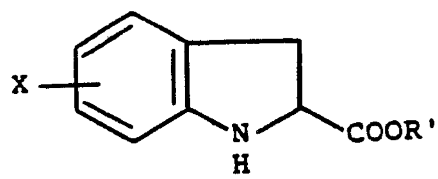

(III)

que l'on fait réagir avec un composé RZ pour obtenir le composé (IV)

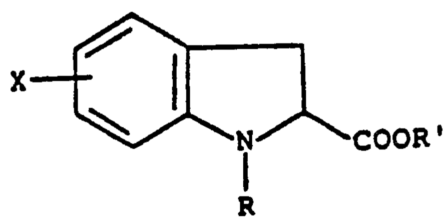

(IV)

que l'on fait réagir avec de l'éthylénediamine en présense de triméthylaluminium,

Z étant un groupe labile, en particulier un atome d'iode ou de brome,

R' étant un radical ($C_{1-4}$)alkyle, et X et R ayant les significations données dans la revendication 1.

7. Procédé de préparation des composés selon la revendication 1, dans la formule (I) desquels R représente un radical phényle éventuellement substitué, procédé caractérisé en ce qu'on fait réagir un acide (V)

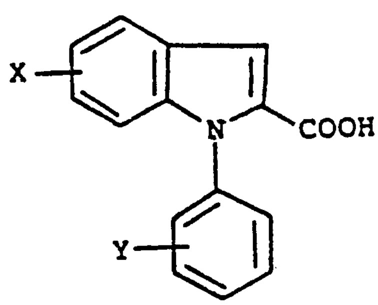

(V)

avec de l'ammoniac en présence de carbonyldiimidazole pour obtenir l'amide (VI)

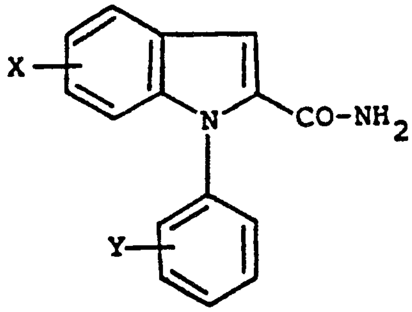

(VI)

que l'on réduit à l'aide d'ammoniac liquide et de sodium, puis on transforme l'amide (VII)

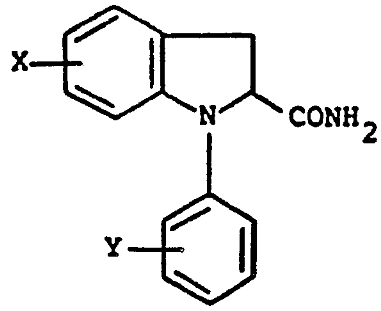

(VII)

en nitrile (VIII)

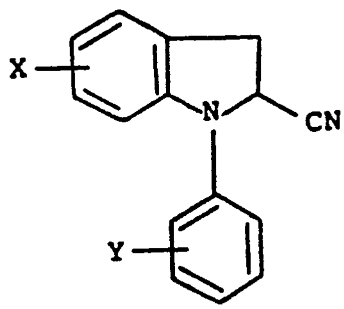

(VIII)

à l'aide de chlorure de p-toluènesulfonyle en présence de pyridine, et on fait réagir le nitrile (VIII) avec de l'éthylènediamine pour obtenir le composé (I), X et Y ayant les significations données dans la revendication 1.

8. Médicament caractérisé en ce qu'il consiste en un composé tel que spécifié dans l'une quelconque des revendications 1 à 5.

9. Composition pharmaceutique caractérisée en ce qu'elle contient un composé tel que spécifié dans l'une quelconque des revendications 1 à 5, en association avec un excipient approprié.

**Revendications pour l'Etat contractant: AT**

1. Procédé de préparation de composés de formule (I), sous forme de racémates ou d'isomères optiquement actifs,

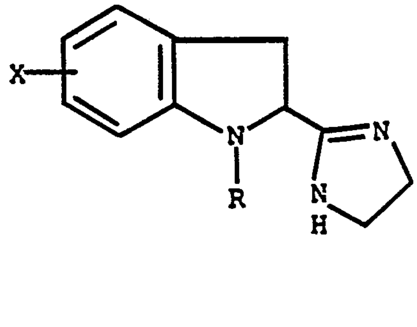

(I)

formule dans laquelle

X est en position 4, 5, 6 ou 7 et représente un atome d'halogène, un radical $(C_{1-4})$alcoxy, un radical $(C_{1-4})$alkyle ou le radical allyloxy, et

R représente un radical $(C_{1-6})$alkyle droit ou ramifié, un radical $(C_{3-6})$cycloalkyl-$(C_{1-4})$alkyle, un radical $(C_{3-6})$alcényle, un radical aryl-$(C_{1-4})$alkyle pouvant porter un ou plusieurs substituants choisis parmi les atomes d'halogène et les radicaux méthyle, méthoxy et méthylènedioxy,

procédé caractérise en ce qu'on hydrogène un ester de formule (II)

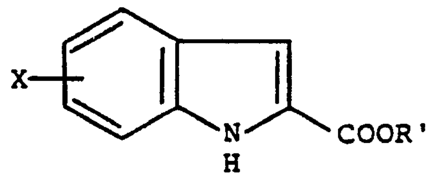

(II)

en ester de formule (III)

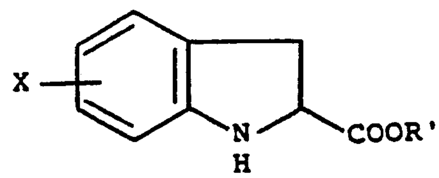

(III)

que l'on fait réagir avec un composé RZ pour obtenir le composé (IV)

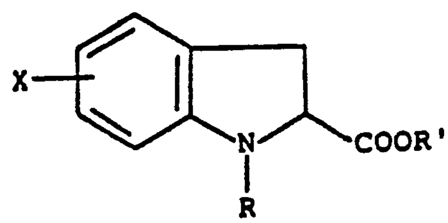

(IV)

que l'on fait réagir avec de l'éthylènediamine en présense de triméthylaluminium,

Z étant un groupe labile, en particulier un atome d'iode ou de brome,

R' étant un radical $(C_{1-4})$alkyle, et X et R ayant les significations données ci-dessus.

2. Procédé de préparation de composés de formule (I), sous forme de racémates ou d'isomères optiquement actifs,

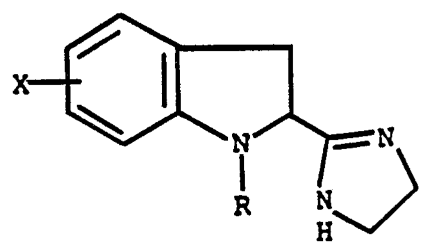

(I)

formule dans laquelle

X est en positions 4, 5, 6 ou 7 et représente un atome d'hydrogène ou d'halogène, un radical $(C_{1-4})$alcoxy, un radical $(C_{1-4})$alkyle ou le radical allyloxy, et

R représente un radical phényle éventuellement substitué, de formule

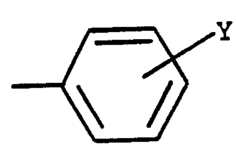

dans laquelle Y représente un atome d'hydrogène ou d'halogène, ou un radical $(C_{1-6})$alkyle droit ou ramifié ou un radical $(C_{1-6})$alcoxy droit ou ramifié,

procédé caractérisé en ce qu'on fait réagir un acide (V)

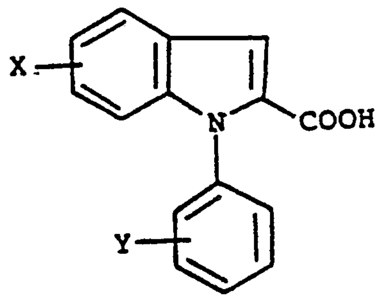

(V)

avec de l'ammoniac en présence de carbonyldiimidazole pour obtenir l'amide (VI)

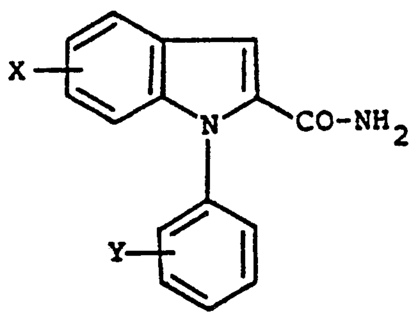

(VI)

que l'on réduit à l'aide d'ammoniac liquid et de sodium, puis on transforme l'amide (VII)

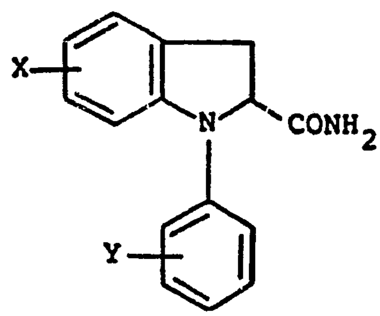

(VII)

en nitrile (VIII)

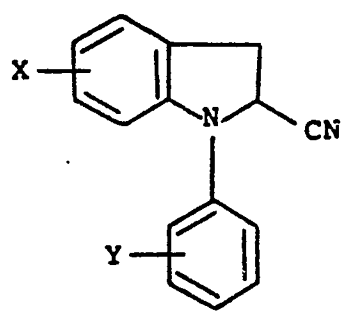

(VIII)

à l'aide de chlorure de p-toluènesulfonyl en présence de pyridine, et on fait réagir le nitrile (VIII) avec de l'éthylènediamine pour obtenir le composé (I), X et Y ayant les significations données ci-dessus.

**Patentansprüche für die Vertragsstaaten: BE CH DE FR GB IT LI LU NL SE**

1. Verbindungen in Form der Racemate oder optisch aktiven Isomeren, dadurch gekennzeichnet, daß sie der allgemeinen Formel (I)

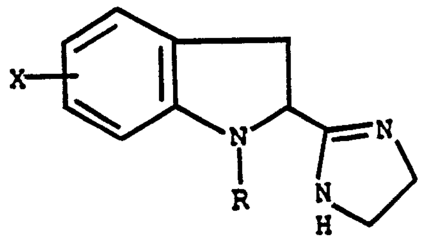

(I)

entsprechen, in welcher X in der Position 4, 5, 6 oder 7 steht und ein Halogenatom, einen $(C_{1-4})$-Alkoxyrest, einen $(C_{1-4})$-Alkylrest oder den Allyloxyrest bedeutet, oder aber, wenn R für einen gegebenenfalls substituierten Phenylrest steht, auch ein Wasserstoffatom darstellen kann, und R einen geradkettigen oder verzweigten $(C_{1-6})$-Alkylrest, einen $(C_{3-6})$-Cycloalkyl$(C_{1-4})$-alkylrest, einen $(C_{3-6})$-Alkenylrest, einen Aryl-$(C_{1-4})$-alkylrest, der einen oder mehrere Substituenten aus der Gruppe der Halogenatome und der Methyl-, Methoxy- und Methylendioxyreste trägt, bedeutet oder aber für einen gegebenenfalls substituierten Phenylrest der Formel

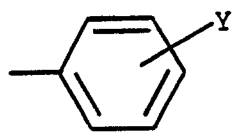

steht, in welcher Y für eine Wasserstoff- oder ein Halogenatom oder einen geradkettigen oder verzweigten $(C_{1-6})$-Alkylrest oder einen geradkettigen oder verzweigten $(C_{1-6})$-Alkoxyrest steht, sowie deren Additionssalze mit pharmazeutisch verwendbaren Säuren.

2. Verbindungen nach Anspruch 1, dadurch gekennzeichnet, daß X ein Chlor- oder Fluoratom oder einen Methyl-, Methoxy- oder Allyloxyrest bedeutet und R für einen Alkyl-, Benzyl-, Allyl oder Cyclopropylmethylrest steht.

3. Verbindungen nach Anspruch 2, dadurch gekennzeichnet, daß X in der Position 4, 6 oder 7 steht.

4. Verbindungen nach Anspruch 1, dadurch gekennzeichnet, daß X ein Wasserstoffatom oder einen Methylrest bedeutet und R für einen Phenyl-, Methylphenyl-, Ethylphenyl-, Methoxyphenyl-, Ethoxyphenyl- oder Propoxyphenylrest steht.

5. Verbindungen nach Anspruch 1, dadurch gekennzeichnet, daß X in der Stellung 4 steht und R eine in Stellung 3 oder 4 substituierte Phenylgruppe bedeutet.

6. Verfahren zur Herstellung der Verbindungen nach Anspruch 1, in deren Formel (I) R für einen Alkyl-, Cycloalkylalkyl-, Alkenyl- oder Arylalkylrest steht, dadurch gekennzeichnet, daß man einen Ester der Formel (II)

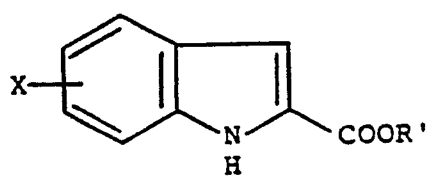

(II)

zu einem Ester der Formel (III)

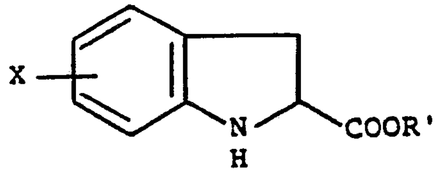

(III)

hydriert, den man mit einer Verbindung RZ reagieren läßt, um die Verbindung (IV)

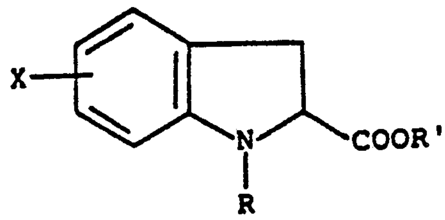

(IV)

zu erhalten, die man ihrerseits mit Ethylendiamin in Gegenwart von Trimethylaluminium zur Reaktion bringt, wobei Z für eine labile Gruppe steht, insbesondere für Iod oder Brom, R' einen ($C_{1-4}$)-Alkylrest bedeutet und X und R die in Anspruche 1 angegebenen Bedeutungen haben.

7. Verfahren zur Herstellung von Verbindungen nach Anspruch 1, in deren Formel (I) R für einen gegebenenfalls substituierten Phenylrest steht, dadurch gekennzeichnet, daß man eine Säure (V)

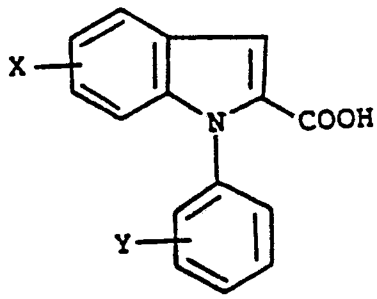

(V)

mit Ammoniak in Gegenwart von Carbonyldiimidazol reagieren läßt, um das Amid (VI)

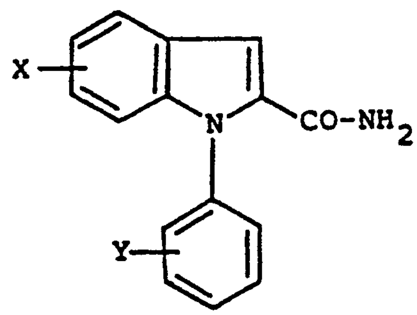

(VI)

zu gewinnen, das man mit Hilfe von flüssigem Ammoniak und Natrium reduziert, worauf man das Amid (VII)

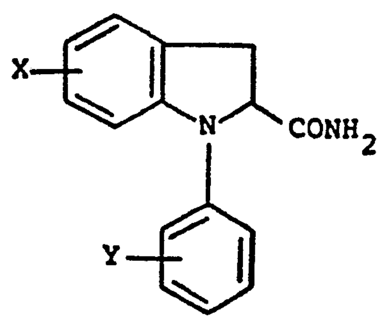

(VII)

mit Hilfe von p-Toluolsulfonsäurechlorid in Gegenwart von Pyridin in das Nitril (VIII)

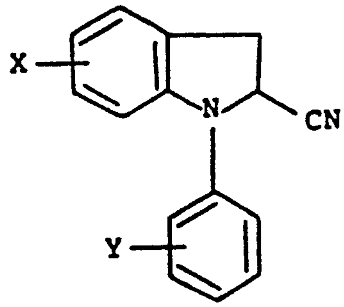

(VIII)

umwandelt und diese Nitril (VIII) mit Ethylendiamin zur Reaktion bringt, um die Verbindung (I) herzustellen, wobei X und Y die in Anspruch 1 angegebenen Bedeutungen haben.

8. Arzneimittel, dadurch gekennzeichnet, daß es aus einer Verbindung besteht, die in einem der Ansprüche 1 bis 5 spezifiert ist.

9. Pharmazeutische Zusammensetzung, dadurch gekennzeichnet, daß sie eine Verbindung nach einem der Ansprüche 1 bis 5 in Kombination mit einem geeigneten Träger enthält.

**Patentansprüche für den Vertragsstaat: AT**

1. Verfahren zur Herstellung von Verbindungen der Formel (I) in Form der Racemate oder optisch aktiven Isomeren

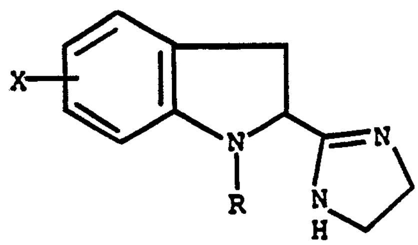

(I)

in welcher X in der Position 4, 5, 6 oder 7 steht und ein Halogenatom, einen $(C_{1-4})$-Alkoxyrest, einen $(C_{1-4})$-Alkylrest oder den Allyloxyrest bedeutet, und R einen geradkettigen oder verzweigten $(C_{1-6})$-Alkylrest, einen $(C_{3-6})$-Cycloalkyl$(C_{1-4})$-alkylrest, einen $(C_{3-6})$-Alkenylrest, einen Aryl-$(C_{1-4})$-alkylrest, der einen oder mehrere Substituenten aus der Gruppe der Halogenatome und der Methyl-, Methoxy- und Methylendioxyreste trägt, bedeutet, dadurch gekennzeichnet, daß man einen Ester der Formel (II)

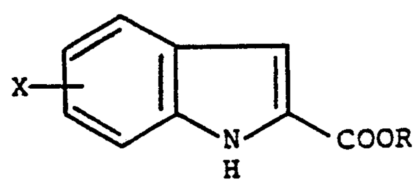

(II)

zu einem Ester der Formel (III)

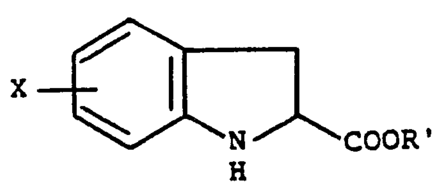

(III)

hydriert, den man mit einer Verbindung RZ reagieren läßt, um die Verbindung (IV)

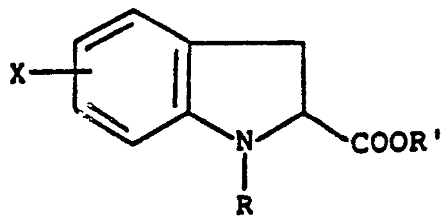

(IV)

zu erhalten, die man ihrerseits mit Ethylendiamin in Gegenwart von Trimethylaluminium zur Reaktion bringt, wobei Z für eine labile Gruppe steht, insbesondere für Iod oder Brom, R' einen $(C_{1-4})$-Alkylrest bedeutet und X und R die oben angegebenen Bedeutungen haben.

2. Verfahren zur Herstellung von Verbindungen der Formel (I) in Form der Racemate oder optisch aktiven Isomeren

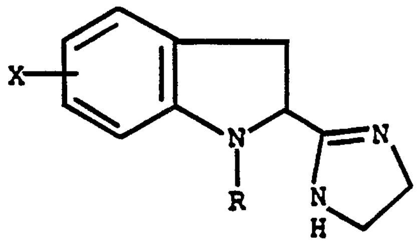

(I)

in welcher Formel X in der Position 4, 5, 6 oder 7 steht und ein Wasserstoff- oder Halogenatom, einen $(C_{1-4})$-Alkoxy, einen $(C_{1-4})$-Alkyl- oder den Allyloxyrest bedeutet und R für einen gegebenenfalls substituierten Phenylrest der Formel

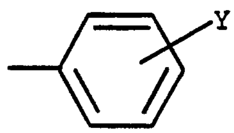

steht, in welcher Y für ein Wasserstoff- oder Halogenatom oder einen geradkettigen oder verzweigten $(C_{1-6})$-Alkylrest oder einen geradkettigen oder verzweigten $(C_{1-6})$-Alkoxyrest steht, dadurch gekennzeichnet, daß man eine Säure (V)

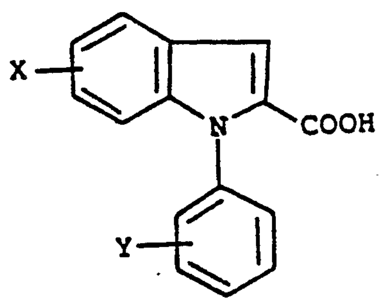

(V)

in Gegenwart von Carbonyldiimidazol mit Ammoniak reagieren läßt, um das Amid (VI)

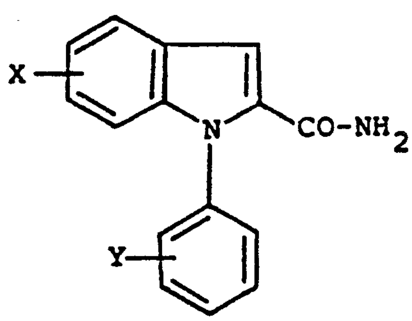

(VI)

zu erhalten, das man seinerseits mit flüssigem Ammoniak und Natrium reduziert, worauf man anschließend das Amid (VII)

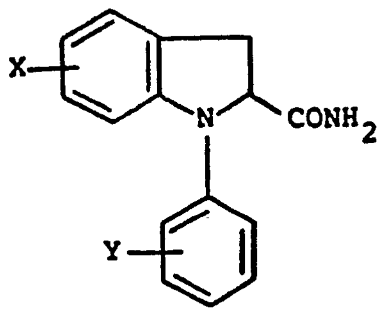

(VII)

mit Hilfe von p-Toluolsulfonsäurechlorid in Gegenwart von Pyridin in das Nitril (VIII)

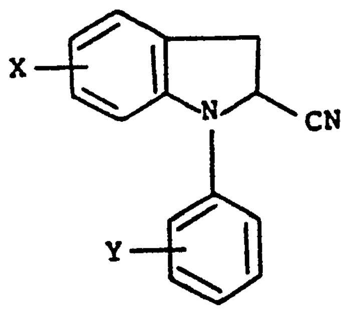

(VIII)

umwandelt, welches Nitril (VIII) man mit Ethylendiamin reagieren läßt, um die Verbindung (I) zu erhalten, wobei X und Y die oben genannten Bedeutungen haben.

**Claims for the Contracting States: BE CH DE FR GB IT LI LU NL SE**

1. Compounds in the form of racemates or optically active isomers, characterized in that they correspond to the general formula (I)

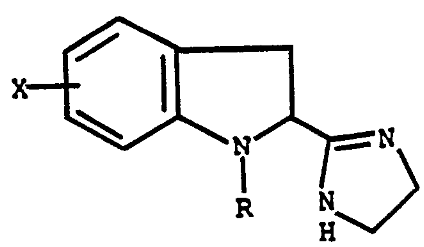

(I)

in which

X is at position 4, 5, 6 or 7 and denotes a halogen atom, a $(C_{1-4})$alkoxy radical, a $(C_{1-4})$alkyl radical or an allyloxy radical, or alternatively, when R denotes a phenyl radical, optionally unsubstituted, X can also denote a hydrogen atom and

R denotes a linear or branched $(C_{1-6})$alkyl radical, a $(C_{3-6})$cycloalkyl-$(C_{1-4})$alkyl radical, a $(C_{3-6})$alkenyl radical, an aryl-$(C_{1-4})$alkyl radical which can bear one or more substituents chosen from halogen atoms and methyl, methoxy and methylenedioxy radicals, or alternatively an optionally substituted phenyl radical of formula

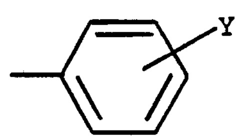

in which Y denotes a hydrogen or halogen atom or a linear or branched $(C_{1-6})$alkyl radical or a linear or branched $(C_{1-6})$alkoxy radical, as well as the addition salts thereof with pharmaceutically acceptable acids.

2. Compounds according to Claim 1, characterized in that X denotes a chlorine or fluorine atom or a methyl, methoxy or allyloxy radical, and R denotes an alkyl, benzyl, allyl or cyclopropylmethyl radical.

3. Compounds according to Claim 2, characterized in that X is at position 4, 6 or 7.

4. Compounds according to Claim 1, characterized in that X denotes a hydrogen atom or a methyl radical, and R denotes a phenyl, methylphenyl, ethylphenyl, methoxyphenyl, ethoxyphenyl or propoxyphenyl radical.

5. Compounds according to Claim 4, characterized in that X is at position 4 and R denotes a phenyl group substituted at position 3 or 4.

6. Process for preparing the compounds according to Claim 1, in the formula (I) of which R denotes an alkyl, cycloalkylalkyl, alkenyl or arylalkyl radical, which process is characterized in that an ester of formula (II)

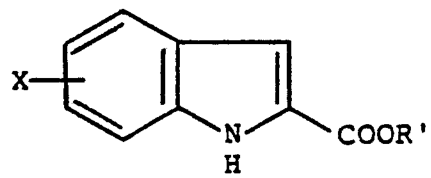

(II)

is hydrogenated to the ester of formula (III)

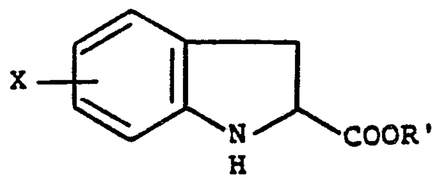

(III)

which is reacted with a compound RZ to obtain the compound (IV)

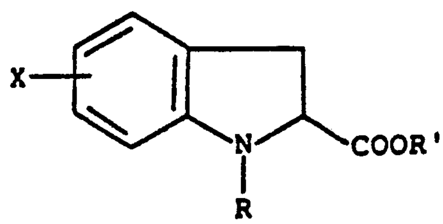

(IV)

which is reacted with ethylenediamine in the presence of trimethylaluminium,

Z being a labile group, especially an iodine or bromine atom,

R′ being a $(C_{1-4})$alkyl radical and X and R having the meanings given in Claim 1.

7. Process for preparing the compounds according to Claim 1, in the formula (I) of which R denotes an optionally substituted phenyl radical, which process is characterized in that an acid (V)

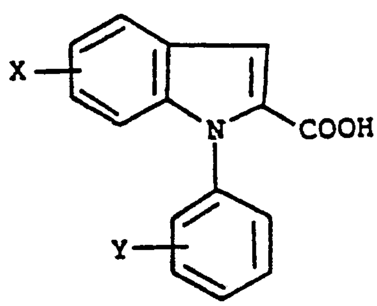

(V)

is reacted with ammonia in the presence of carbonyldiimidazole to obtain the amide (VI)

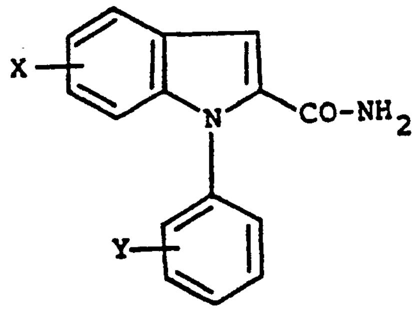

(VI)

which is reduced using liquid ammonia and sodium, the amide (VII)

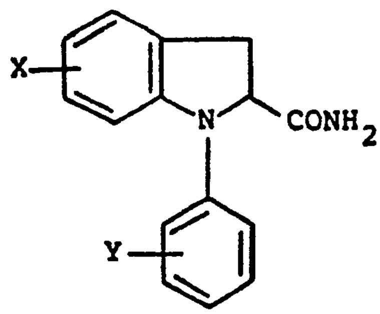

(VII)

is then converted to the nitrile (VIII)

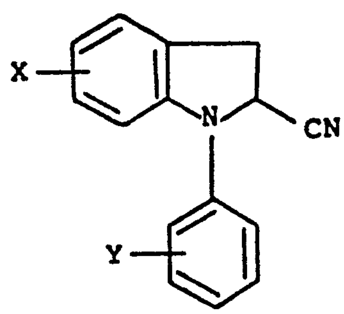

(VIII)

using p-toluenesulphonyl chloride in the presence of pyridine, and the nitrile (VIII) is reacted with ethylenediamine to obtain the compound (I), X and Y having the meanings given in Claim 1.

8. Drug, characterized in that it consists of a compound as specified in any one of Claims 1 to 5.

9. Pharmaceutical composition, characterized in that it contains a compound as specified in any one of Claims 1 to 5, in combination with a suitable excipient.

**Claims for the Contracting State: AT**

1. Process for preparing compounds of formula (I), in the form of racemates or optically active isomers,

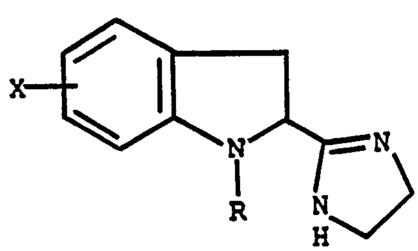

(I)

in which formula

X is at position 4, 5, 6 or 7 and denotes a halogen atom, a $(C_{1-4})$alkoxy radical, a $(C_{1-4})$alkyl radical or an allyloxy radical, and

R denotes a linear or branched ($C_{1-6}$)alkyl radical, a ($C_{3-6}$)cycloalkyl-($C_{1-4}$)alkyl radical, a ($C_{3-6}$)alkenyl radical, or an aryl-($C_{1-4}$)alkyl radical which can bear one or more substituents chosen from halogen atoms and methyl, methoxy and methylenedioxy radicals,

which process is characterized in that an ester of formula (II)

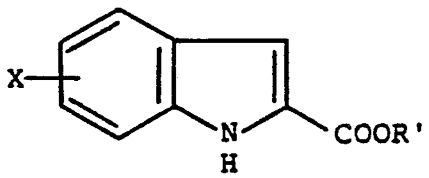

(II)

is hydrogenated to the ester of formula (III)

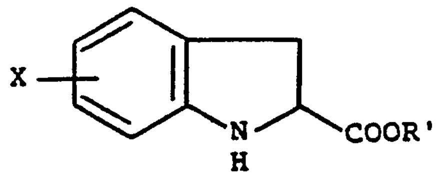

(III)

which is reacted with a compound RZ to obtain the compound (IV)

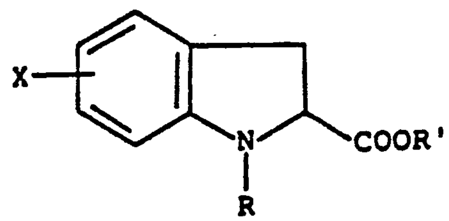

(IV)

which is reacted with ethylenediamine in the presence of trimethylaluminium,

Z being a labile group, especially an iodine or bromine atom,

R′ being a ($C_{1-4}$)alkyl radical, and X and R having the meanings given above.

2. Process for preparing compounds of formula (I), in the form of racemates or optically active isomers,

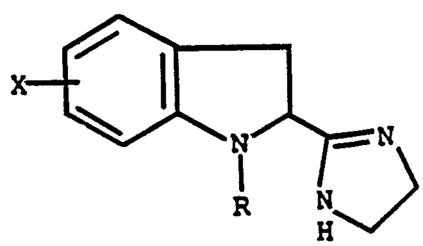

(I)

in which formula

X is at position 4, 5, 6 or 7 and denotes a hydrogen or halogen atom, a ($C_{1-4}$)alkoxy radical, a ($C_{1-4}$)alkyl radical or an allyloxy radical, and

R denotes an optionally substituted phenyl radical, or formula

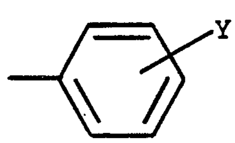

in which Y denotes a hydrogen or halogen atom or a linear or branched ($C_{1-6}$)alkyl radical or a linear or branched ($C_{1-6}$)alkoxy radical, which process is characterized in that an acid (V)

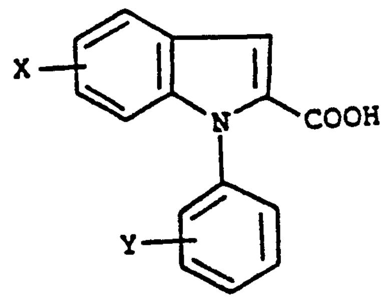

(V)

is reacted with ammonia in the presence of carbonyldiimidazole to obtain the amide (VI)

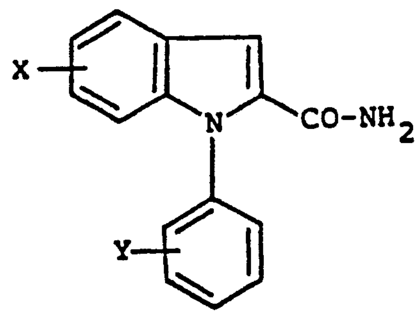

(VI)

which is reduced using liquid ammonia and sodium, the amide (VII)

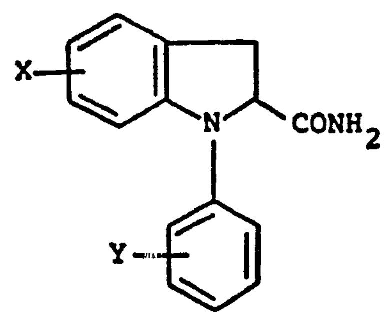

(VII)

is then converted to the nitrile (VIII)

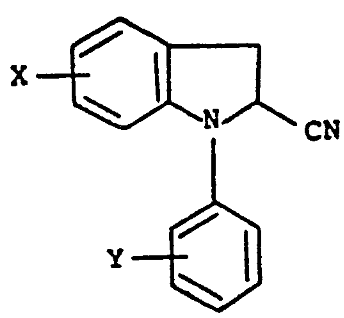

(VIII)

using p-toluenesulphonyl chloride in the presence of pyridine, and the nitrile (VIII) is reacted with ethylenediamine to obtain the compound (I), X and Y having the meanings given above.